# EUROPEAN PATENT APPLICATION

(11) **EP 1 114 651 A1**
(43) Date of publication of application: **11.07.2001**
(21) Application number: 00944055.3
(22) Date of filing: 13.07.2000
(51) Int. Cl.: A61M 25/00, A61M 25/02

(54) **PROTECTOR FOR CATHETERS OF CLINICAL USE**

(30) Priority: 16.07.1999 ES 9901910 U
(71) Applicant: Martinez Perez, Teresa, 30009 Murcia (ES)
(72) Inventor: MARTINEZ PEREZ, Teresa, E-30009 Murcia (ES); HERNANDEZ PEREZ, M., José, E-30009 Murcia (ES)
(74) Representative: Morgades Manonelles, Juan Antonio
(86) International application number: ES0000249
(87) International publication number: WO0105461

(57) **Abstract**

"PROTECTOR FOR CATHETERS FOR CLINICAL USE"

It is constituted by a pouch manufactured of an sterile material suitable for offering a cushioning for the catheter and becoming a barrier for the environmmental microorganisms. The pouch is adpated for lodging inside any catheter type and features an adhesive strip near its mouth as an extension of its anterior face. The strip constitutes a means for the fixation of the protector to the patient's body and a means of closing and sealing of the catheter s pouch itself. Said strip is a self-adhesive strip comprising protecting sheets of waxed or siliconated paper or the like, which are removed in order to its fixation to the patient's body.

## Description

The present invention relates to a protector which has been specially designed for catheters for clinical use, for achieving in a quick and simple way estabilishing the suitable protection degree for the catheter and the resulting comfort for the patient during the time periods in which said catheter is not operative. Also, the proposed invention makes easier the manipulation of same by the sanitary personnel.

The protector consist in a single use element, duly sterilized, which fully covers the catheter in the not operative position of same, avoiding its contamination with microorganisms which may be harmful for the patient and which would have access to his blood system through the catheter itself, in case that it was not suitably protected, making easier at the same time the different treatments needed by the patient.

When a patient is the bearer of an permanent or semi-permanent endoveinous catheter for his different treatments (peripheric veins, catheters for haemodialysis, etc,), the care of said catheter by the sanitary personnel presents difficulties since the material existing in the market is not foreseen for that use.

Since the catheter must be aseptically insulated in order to avoid the access through same of the microorganisms on the environment, there are actually employed bulky adhesive dressings and gauzes which mean a problem basically centered in two aspects, both requiring special skills and dexterity of the sanitary personnel, who must cut gauzes, remove adhesives, etc., and it is an operation frequently repeated, which means also disconfort to the patient and skin rashes, to which must be added the important risk that, when removing the dressing, the catheter may result uncoupled from the vein where it was implanted.

The protector for catheters for clinical use that is proposed by the invention solves in a fully satisfying manner the above problems, in the diferent aspects disclosed, allowing an easy and quick implant and withdrawal of same, with a minimum of disconfort to the patient and without any risk of uncoupling the catheter.

In order to that, and in a more definite way, said protector is incorporated as an sterile pouch, of a suitable material for placing the catheter keeping it insulated from the skin and resulting in a barrier to the environmental microorganisms, of variable dimensions according to the type of catheter to which is intended, with the special feature that said pouch ends at its mouth in an adhesive strip provided with protection sheets of waxed or siliconated paper or the like, in such a way that, in order to implant the protector, it s enough to couple the pouch for keeping the catheter lodged inside, up to a limit situation in which the adhesive strip, after removing the protecting sheets, is fixed to the body of the patient acting as a fixation means of the protector as a whole and a sealing means of the pouch s mouth.

Obviously, in order to withdraw the protector it s enough to remove the adhesive strip, in order that the puoch may be easily eliminated simply displacing it axially with respect to the catheter.

In order to complete the ongoing description and for helping to a better understanding of the invention features, according with an example of a practical preferred incorporation of same, there are joined as an integral part of said description, a set of drawings where, in an illustrative but not limitative way, there is depicted what follows:
Figure 1 shows in a perspective view a protector for catheters for clinic use incorporated acording to the object of the present invention.
Figure 2 shows a lengthwise section of the protector of Figure 1, as per line A-B in said figure.
Figure 3 shows, finally and also as per a perspective view, an practical example of the use of the protector.

Looking at these Figures there can be seen that the protector proposed by the invention is incorporated in a sort of pouch (1), elongated, of an sterile material and adequate for estabilishing a perfect cushioning for the catheter and become a barrier to the microorganisms, a pouch (1) which mouth (2) is placed corresponding with one of its ends and which dimensions either in length or width will be the suitable ones for the type of the catheter (3) to be received inside.

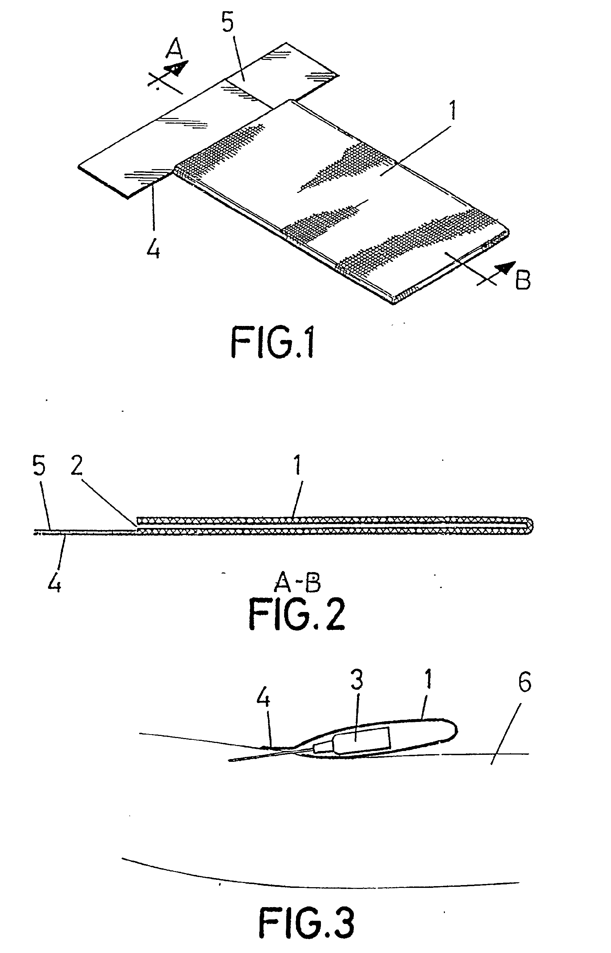

The front face of the pouch (1), the one which will be its outer wall after installing the protector, extends over its mouth (2) in an adhesive strip (4), transversely placed, like, for example, the classic self-adhesive strips provided with protecting sheets (5), easily removable, the commonly named tiritas , in such a way that after the catheter (3) is implanted inside the pouch (1), as can be seen in Figure 2 and with either a previous or later removal of the protecting sheets (5), the adhesive strip (4) is fixed to the user s body (6) fullfilling the double task of, in a way, positionally stabilizing the pouch (19 with regard to the body (6) of the user and, in the other way, close or seal said pouch with the catheter (1) inside.

Obviously, the protector will be marketed packaged in an airtight way, keeping the sterile characteristics up to the moment of the use, having foreseen for same, as earlier discussed, the nature of a single use, therefore, after its single use it will be eliminated and substituted with a new one, in order to insure its real protecting effect.

## Claims

1. "PROTECTOR FOR CATHETERS FOR CLINICAL USE" characterized in that it consists in a sort of pouch, of an sterile material suitable for estabilishing a perfect cushioning for the catheter and constituting a barrier for the environmental microorganisms, a pouch with dimensions suitable for lodging inside the specific type of catheter intended, with the special feature that said pouch incorporates at the level of the mouth, and as an extension of its anterior face, an adhesive strip, transversely elongated, constituting both the means for the fixation of the protector to the patient s body as well as the means for closing and sealing the pouch itself.

2. "PROTECTOR FOR CATHETERS FOR CLINICAL USE" as per Claim 1 characterized in that said adhesive strip is a self-adhesive strip provided with protecting sheets of waxed or siliconated paper or the like, which may be eliminated at the moment of its fixation to the patient s body.
